Europäisches Patentamt

(19) European Patent Office

Office européen des brevets

(11) Publication number : **0 516 278 A1**

# EUROPEAN PATENT APPLICATION

(21) Application number : 92303689.1

(22) Date of filing : 24.04.92

(51) Int. Cl.$^5$ : **A61K 37/24,** A61K 31/00, A61K 31/565, A61K 31/44

(30) Priority : 26.04.91 US 691949
26.04.91 US 691947

(43) Date of publication of application :
02.12.92 Bulletin 92/49

(84) Designated Contracting States :
CH DE FR GB IT LI NL

(71) Applicant : MERCK & CO. INC.
126, East Lincoln Avenue P.O. Box 2000
Rahway New Jersey 07065-0900 (US)

(72) Inventor : Elbrecht, Alex
65 Maple Street
Watchung, NJ 07060 (US)
Inventor : Smith, Roy G.
528 Forest Avenue
Westfield, NJ 07090 (US)

(74) Representative : Thompson, John Dr. et al
Merck & Co., Inc. European Patent
Department Terlings Park Eastwick Road
Harlow, Essex CM20 2QR (GB)

(54) **Use of anti-mullerian hormone for reversal of female sexual phenotype in poultry.**

(57)    Fertilized poultry embryos are treated with fowl antimullerian hormone which may function as an aromatase inhibitor and may also have a trophic effect on developing gonads. Fertilized poultry embryos are also treated with steroid biosynthesis inhibitors or antagonists which prevents the conversion of androgens to estrogens, and anti-mullerian hormone (AMH). By blocking the production of estrogens and enhancing the gonads the genotypic female is converted into a phenotypic male. The phenotypic conversion of females to males gives the treated birds the advantage of male growth characteristics. A single administration of the combination prior to about day 9 of embryonic incubation results in an irreversible change in sexual phenotype which results in enhanced weight gain and feed efficiency.

EP 0 516 278 A1

BACKGROUND OF THE INVENTION

Males of commercially important poultry species gain weight faster during the first eight weeks after hatching than do females of the same species, Nixey, 7th Eur. Poultry Conf., Vol. 2, Larbier, ed., pg. 671-679 (1988). This weight gain advantage allows male birds to reach a marketable weight before females. A uniform weight gain of both male and female birds would have a significantly positive economic impact on broiler production for all important species.

Sex reversal in female chickens has been accomplished by early grafting of female chicken embryos with embryonic testes, Stoll, et al., Gen. Comp. Endocrinol. 41:66-75 (1980). The grafted testes acted during the normal period of gonadal differentiation, between 6 and 9 days of incubation, and the changes were permanent as evidenced by male characteristics in the adult birds. The sex reversed birds possess two testes associated with normally differentiated male excretory ducts and their Mullerian ducts have regressed. The development of male sex characteristics, in female genotypes, such as external features, behavior and complete spermatogenesis is evidence that these cocks have endocrine and exocrine capabilities similar to those of genotypic males.

The conversion of female genotypic embryos to male phenotype following embryonic testis grafting appears to depend on a substance or substances secreted into the blood of the female host by the grafted testis, Rashedi et al., Biol. Reprod. 29: 1221-1227 (1983). The authors propose that the masculinizing property of the grafted testes depends on antimullerian hormone (AMH), Mullerian-inhibiting substance (MIS), which is responsible for the regression of the Mullerian ducts during normal differentiation.

Sexual differentiation of birds is dependent upon complex physiological control of an anatomically complicated urogenital system. The female bird has an ovary and a persistent Mullerian duct on the left side and a small ovotestis and no Mullerian duct on the right compared to the male, which has bilateral testes and no Mullerian ducts. It has been observed that chick gonads, female as well as male, produce MIS which may cause regression of the right Mullerian duct in the female, Huston et al., J. Pediatr Surg. 16, 822-827 (1981). This did not answer the question of why the left duct in the female does not regress despite ovarian MIS production. It has been postulated that estrogen may protect the left duct, since exogenous estrogens cause feminization of the male chick embryo, with regression of the Mullerian ducts, Rahil and Narbaitz Gen. Comp. Endocrinol. 18: 315-318 (1972). Studies with antiestrogens, such as tamoxifen and LY117018, had little effect on the female Mullerian ducts unless given in high doses or with added testosterone, Huston et al., Gen. Comp. Endocrinol. 57: 88-102 (1985). Huston et al. also observed that a postulated aromatase inhibitor, norethindrone, caused partial regression of the upper end of the left Mullerian duct as well as complete loss of the lower ends of both ducts in the female. The authors suggested that the steroid environment is a critical factor in the response of the Mullerian ducts to MIS and that estrogen blockage may allow endogeneous MIS in the ovary to induce partial regression of the Mullerian ducts in the female chick embryo. None of the references relating to sexual differentiation of birds describe complete phenotypic transformation from females to males.

Studies using mammalian systems suggests that MIS directly modulates aromatase activity to affect gonadal differentiation. Mammalian MIS, produced only in the testes, induces the regression of the Mullerian ducts of the male fetus. Exposure of ovine fetal ovaries to MIS induced an endocrine sex reversal resulting in the release of testosterone instead of estradiol due to the suppression of aromatase activity, Vigier et al., Proc. Natl. Acad. Sci. USA 86: 3684-3688 (1989). The avian and mammalian responses to MIS and its role in differentiation are noticeably different and data gained from mammalian experiments should not be extrapolated to birds, Hutson et al., Reproduct. Physiol. IV, In Inter. Rev. Physiol. 27: 177-223 (1983).

OBJECTS OF THE INVENTION

It is, accordingly, an objective of the present invention to provide a means of converting genotypic female birds into phenotypic male birds. Another object is to treat bird embryos with anti-mullerian hormone to prevent the conversion of testosterone to estradiol and to enhance the maturation of the gonads. A further object is to treat bird embryos with antimullerian hormone to convert female genotypic birds into male phenotypic birds. Another object is to treat bird embryos with steroid biosynthesis inhibitors or antagonists to prevent the conversion of testosterone to estradiol, and AMH to enhance the development of gonads into testes. A further object is to treat bird embryos with an aromatase inhibitor plus AMH to convert female genotypic birds into male phenotypic birds. Another object is to convert genotypic female birds into phenotypic male birds so that following hatching all birds of a flock will gain weight at the rate of males or that the converted males will gain weight at a rate greater than non-treated females. A further object is to obtain phenotypically male birds with a female genotype. Another object is to be able to irreversibly change the sexual phenotype of birds by treating a bird embryo with steroid biosynthesis inhibitors or antagonists plus AMH prior to about day 9 of embryonic development.

SUMMARY OF THE INVENTION

Fertilized poultry embryos are treated with fowl antimullerian hormone which may function as an aromatase inhibitor and may also have a trophic effect on developing gonads. Fertilized poultry embryos are also treated with steroid biosynthesis inhibitors or antagonists which prevents the conversion of androgens to estrogens, and anti-mullerian hormone (AMH). By blocking the production of estrogens and enhancing the gonads the genotypic female is converted into a phenotypic male. The phenotypic conversion of females to males gives the treated birds the advantage of male growth characteristics. A single administration of the combination prior to about day 9 of embryonic incubation results in an irreversible change in sexual phenotype which results in enhanced weight gain and feed efficiency.

## DETAILED DESCRIPTION OF THE INVENTION

The present invention relates to the conversion of a genotypic female fowl to a phenotypic male fowl. Fowl is defined herein as wild or domesticated gallinaceous birds that serve as a source of meat and that include, but are not limited to, commercially important birds such as chickens, turkeys, ducks, geese, guinea fowl, pheasants, pigeons, peafowl and quail. Fowl as used herein will also include Poultry, all domesticated birds kept for meat.

Fertilized embryos of any domesticated gallinaceous bird, with chicken, goose, duck and turkey being preferred, are treated with an aromatase inhibitor to prevent the conversion of testosterone to estradiol plus AMH to allow development of testes in female fowl. Aromatase is an enzyme complex incorporating a NADPH-cytochrome c-reductase and a cytochrome $P_{450}$ component which mediates the conversion of androgens to estrogens, Bellino, J. Steroid Biochem. 17: 261-270 (1982). The reaction is believed to involve three hydroxylation steps, two at the C-19 position (Meyer, Biochem. Biophys. Acta 17: 441-442 [1955]; Morato et al., Biochem. Biophys. Res. Comm. 6: 334-338 [1961]) and one at C-2 (Hahn and Fishman, J. Biol. Chem. 259: 1689-1694 [1984]; Brodie et al., J. Am. Chem. Soc. 91: 1241-1242 [1969]) which result in the conversion of the A ring of the androgen molecule to an aromatic ring. Since aromatization is a unique reaction in the biosynthesis of steroids, specific inhibitors should not cause deprivation of other essential steroids. The inhibition or blocking of the conversion of androgens (testosterone, androstenedione) to estrogens (estradiol, estrone) results in an accumulation of androgens (testosterone, androstenedione) which may allow the gonad in genetic females to differentiate into a testis and prevents the regression of the right gonad resulting in the production of phenotypic male birds from genotypic female birds. An aromatase inhibitor as defined herein is any steroidal or non-steroidal compound which prevents the conversion of androgens to estrogens. The compounds include substrate analogues of androstenedione, testosterone or other steroidal substances involved in the aromatase pathway, Henderson, J. Steroid Biochem. 27: 905-914 (1987). Non-steroidal compounds that block aromatase activity are also included within the scope of the invention. These non-steroidal compounds include compounds or analogues that can bind to the enzymatic active site of aromatase and inhibit enzymatic activity. Non-steroidal or steroidal compounds will also include compounds or analogues that bind to the enzyme at a site away from the enzymatic site and cause a structural change in the enzyme which results in a loss of enzymatic activity. Aromatase inhibitors further include non-steroidal compounds that interfere with cytochrome $P_{450}$ mediated hydroxylations such as those described by Brodie et al., J. Steroid Biochem. 27: 899-903 (1987).

The following compounds are known aromatase inhibitors as disclosed by the associated reference. The reference will also directly contain a method for making the compound or will direct one who wishes to use the compound to a method for producing the compound. The aromatase inhibitors of the instant invention include, but are not limited to, the following compounds: 6-[(1H-imidazol-1-yl)phenylmethyl]-1methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, 2,2-[5-dideuterio(1H-1,2,4-triazol-1-yl)methyl-1,3-phenylene]di(2-trideuteriomethyl-3,3,3-(trideuteriopropiononitrile), and 2,2-[5-dideuterio(1H-1,2,4-triazol-1-ylmethyl-1,3-phenylene)di(2-methylpropiononitrile) as disclosed in European Patent Application, Publication No. 296,749; 1,1-dimethyl-8-(1H-1,2,4-triazol-1-ylmethyl)-2(1H)-naphtho[2,1-b]furanone, 1,2-dihydro-1,1-dimethyl-2-oxo-8-(1H-1,2,4-triazol-1-ylmethyl)naphtho-[2,1-b]furan-7-carbonitrile, 1,2-dihydro-1,1-dimethyl-2-oxo-8-(1H-1,2,4-triazol-1-ylmethyl)naphtho[2,1-b]furan-7-carboxamide, and 1,2-dihydro-1,1-dimethyl-2-oxo-8-[di(1H-1,2,4-triazol-1yl)methyl]naphtho[2,1-b]furan-7-carbonitrile as disclosed in European Patent Application, Publication No. 316,097; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)1-(1,2,4-triazol-1-yl)-2-(1,2,4-triazol-1-ylmethyl)butan-2-ol, 2-(4-chloro-α-fluorobenzyl)-1,3-di(1,2,4-triazol-1-yl)propan-2-ol, 2-(4-chlorobenzyl)-1,3-bis(1,2,4-triazol-1-yl)propane, 4-[2-(4-chlorophenyl)-1,3-di(1,2,4-triazol-1-ylmethyl)ethoxymethyl]-benzonitrile, 1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)- 1,3-di(1,2,4-triazol-1-yl)propan-2-ol, 1-(4-cyanobenzyl)-2-(2,4-difluorophenyl)1,3-di(1,2,4-triazol-1-yl)-propan-2-ol and 2-(4-chlorophenyl)-1-phenyl-1,3-di(1,2,4-triazol-1-yl)-propan-2-ol as described in European Patent Application, Publication No. 299,684; 5-bis(4-chlorophenyl)methylpyrimidine as disclosed in U.S. Patent No. 4,762,836; α,α-bis(4-chlorophenyl)-2-pyrazinemethanol as described in U.S. Patent No. 4,764,376; N-(2,4-difluorophenyl)-N-benzyl-3-pyridinemethanamine and N-(2-chlorophenyl-α-(4-fluorophenyl)-3-pyridinemethanamine as disclosed in U.S. Patent No. 4,744,251; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol as disclosed in U.S. Patnet No. 4,757,076; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine and disclosed in U.S. Patent No. 4,728,645; 5-[(1H-imidazol-1-yl)phenylmethyl]-2-methyl-1H-benzimidazole and 5-[(3-chlorophenyl)(1 H-imidazol-1-yl)methyl]-1H-benzimidazole as disclosed in European Patent Application, Publication No. 260,744; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl)stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)stilbene-4-carbonitrile, (Z)-α-(imidazol-1-ylmethyl)stilbene4,4'-dicarbonitrile,(Z)-α-(5-methylimidazol-1-ylmethyl)stilbene-4,4'-dicarbonitrile, and (Z)-2-[2-(4-cyanophenyl)-3-(1,2,4-triazol-1-yl)propenyl]pyridine-5-carbonitrile as disclosed in European Patent Application, Publication No. 299,683; (1R* ,2R*)-6-fluoro-2-(4-fluorophenyl)-1,2,3,4-tetrahydro-1-(1H-1,2,4-triazol-1-ylmethyl)naphthalene, (1R* ,2R*)-6-fluoro-2-(4-fluorophenyl)-1,2,3,4-tetrahydro-1-(1H-imidazolylmethyl)-naphthalene, (1R* ,2R*)-and (1R* ,2S*)-2-(4-fluorophenyl)-1,2,3,4-tetrahydro-1-(1H-1,2,4-triazol-1-ylmethyl)naphthalene-6-carbonitrile, (1R* ,2R*)-and (1R* ,2S*)-2-(4-fluorophenyl)-1,2,3,4-thetahydro-1-(1H-imidazolylmethyl)naphthalene-6-carbonitrile, (1R* ,2R*)-and (1R*,2S*)-1,2,3,4-tetrahydro-1-(1H-1,2,4-triazol-1-ylmethyl)naphthalene-2,6-dicarbonitrile, (1R* ,2R*)and (1R* ,2S*)-1,2,3,4-tetrahydro-1-(1H-imidazol-1-ylmethyl)naphthalene-2,6-dicarbonitrile, and(1R* ,2S*)-2-(4-fluorophenyl)-1,2,3,4-tetrahydro-1-(5-methyl-1H-imidazolylmethyl)naphthalene-6-carbonitrile as disclosed in European Patent Application, Publication No. 281,283; 8-chloro-5-(4-chlorophenyl)-5H-indeno[1,2-d]pyrimidine as disclosed in U.S. Patent No. 4,769,378; 5-bis (4-chlorophenyl) methylpyrimidine as disclosed in U.S. Patent No. 4,762,836; 10-(2-propynyl)estr-4-ene-3,17-dione as disclosed in U.S. Patent No. 4,322,416; 6-[(4-chlorophenyl)(1H-1,2,4-triazol-1-yl)methyl]-1-methyl-1H-benzotriazole as described in European Patent Application, Publication No. 293,978; 1-methylandrosta-1,4-dien-3,17-dione as disclosed in U.S. Patent No.4,591,585; 3-ethyl-3-(4-pyridyl)piperidine-2,6-dione as disclosed in British Patent GB 2,151,226; 4-hydroxyandrostene-3,17-dione as disclosed in U.S. Patent No. 4,500,523; 4-(5,6,7,8-tetrahydroimidazo[1,5-a]pyridin-5yl)benzonitril as disclosed in U.S. Patent No. 4,728,645; 6-methyleneandrosta-1,4-diene-3,17-dione, 4-aminoadrostan-1,4,6-trien-3,17-dione and 4-aminoandrosta-4,6-diene-3,17-dione as disclosed in U.S. Patent No. 4,757,061; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole as disclosed in European Patent Application, Publication No. 260,744; 10β-thiiranylestr-4-ene-3,17-dione and 10β-oxiranylestr-4-ene-3,17-dione as disclosed in J. Organ. Chem. 53: 5947-5951 (1988); 3-ethyl-3-(4-pyridyl)piperidine-2,6-dione as described in U.S. Patent No. 4,668,689; 3-(4-aminophenyl)-3-ethyl-pyrrolidine-2,5-dione as disclosed in J. Med. Chem. 29: 520-523 (1986); 1-(7-carboxyheptyl)-imidazole as described in U.S. Patent No. 4,320,134; 1,1-dimethyl-8-(1H-1,2,4-triazol-1-ylinethyl)-2 (1H)-naphtho[2,1-b]furanone (1a) as disclosed in European Patent Application, Publication No. 316,097; ± 5-(p-cyanophenyl)-5, 6, 7, 8-tetrahydroimidazo [1, 5-a] pyridine hydrochloride as disclosed in U.S. Patent No. 4,617,307; 1,4,6-androstatriene-3,17-dione disclosed in Biochem. Pharmac. 31: 2017-2023 (1982) and other compounds well known in the art of aromatase inhibition and cancer therapy such as bis-(p-cyanophenyl)-imidazo-1-yl-methane hemisuccinate and pharmaceutically acceptable derivatives, acid addition salts and possible stereochemically isomeric forms thereof, if and where appropriate. The invention is also intended to include any biologically active equivalents of an aromatase inhibitor as described above.

Regression of Mullerian ducts (Mds) in males is dependent upon a cell regulating protein secreted by the embryonic testes, Jost, Recent Prog. Horm. Res. 8: 379-418 (1953). This protein was originally referred to as Mullerian Inhibitory Substance (MIS) but has subsequently been termed Anti-Mullerian Hormone (AMH). Mammalian AMH is a 140 kDa glycoprotein consisting of identical subunits and secreted by the Sertoli cells testes, Picard and Josso, Biomedicine 25: 147-150 (1976; Bidzik et al., Cell 34: 307-314 (1983). Genes for bovine and human AMH have been cloned, Cate, et al.,Cell 45: 685-698 (1986). Avian AMH has been isolated and purified by Teng et al., Devel. Biol. 123: 245-254 (1987) and appeears to be somewhat larger than its mammalian counterpart.

An important distinction between mammalian and avian AMH is that although avian AMH (chick) is capable of causing regression of mouse Mds, Mammalian AMHs (rat or calf do not have the same effect on avian Mds, Hutson et al., J. Pediatr. Surg. 16: 822-827 (1981). Thus it appears that avian AMH and/or its receptor possess unique properties but still elicit the common response of Md regression. The mechanisms involved in regression have not been identified.

A further difference between mammalian and avian systems is that both avian embryonic ovaries secrete AMH. This leads to regression of the right duct, but the left appears to be insensitive to AMH and develops to form the adult oviduct. It is believed that high local levels of estrogens produced by the left ovary protect the left Md from the effects of AMH.

AMH appears to have at least two other effects on the development of the male phenotype. It acts as an aromatase inhibitor in vitro, Vigier et al., Proc. Natl. Acad. Sci. USA 86: 3684-3688 (1989), suggesting that it might be involved in the regulation of gonadal steroid levels during development. Inhibition of the aromatase enzyme would prevent the production of estrogens, stopping biosynthesis at the level of androgens. AMH also appears to have a trophic effect on testes. The gonads of female transgenic mice expressing an exogenous AMH gene contain structures that resemble seminiferous tubules seen in normal male testes, Behringer et al., Nature 345: 167-170 (1990).

Although AMH can act as an aromatase inhibitor in vitro in is not known at this time whether it preforms this function in vivo. AMH in combination with one or more aromatase inhibitors will permit the use of lower levels of each to cause inhibition of aromatase enzyme activity. This will result in increased specificity by avoiding any secondary activities that might be the result of higher concentrations. The additional trophic effects that AMH has on testes development is also required for proper gonadal development and sex reversal.

During the first week of incubation the chick embryonic gonads are bipotential. Differentiation of the gonads is programmed by genetic factors but can be influenced by treatment with exogenous substances. The pleiotropic effects of AMH on development of the male phenotype would suggest that treatment of female chick embryos with AMH, or factors with AMH-like activity, would result in female-to-male sex-reversal.

Fowl testicluar anti-mullerian hormone is purified from gallinaceous bird testes, with chicken being preferred, isolated from about 8 week of male chicks following the procedure of Teng et al., Develop. Biol. 123: 245-254 (1987). The eight-week-old chickens are killed and the testes are removed immediately. About seen to about twenty grams of testes tissue is obtained from about 25 to about 35 chicks and is pooled, stripped of adhering fat, and connective tissue. The tissue is weighed and finely minced with a curved iris scissors. All the subsequent isolation and purification procedures are performed under sterile conditions in the shortest time necessary.

The minced tissue is suspended in modified Dulbecco's medium containing penicillin and streptomycin and in the presence of benzamidine (about 5 mM). The suspension is transferred to a flask and cultured at about 37°C with constant shaking at about 100 rpm in a New Brunswick Model 25 water bath shaker for about 90 min. The suspension is centrifuged at about 40,000 g for about 60 min at about 4°C. The supernatant is precipitated with powdered ammonium sulfate overnight (at about 40% saturation) at about 4°C and collected by centrifugation at about 8,000 g for 30 min at about 4°C. The precipitate is redissolved in sodium phosphate buffer containing about 10 mM sodium phosphate, about 50 mM NaCl, about 1 mM EDTA, and about 0.03% NaN$_3$ at pH 8.0.

The following sequences for AMH isolation are carried out according to the methods of Budzik et al, Cell 21: 909-915 (1980) and Budzik et al., Cell 34: 307-314 (1983). The redissolved 40% ammonium sulfate precipitated pellet was desalted by passage through a Sephadex G-25 column. The first peak (G-25 pl) containing crude MIS and other proteins is collected and concentrated to about 15 ml (with a protein concentration of about 8.5 mg/ml). This concentrated fraction is passed through a DEAE Bio-Gel A column (2.5 x 30 cm) using the same phosphate buffer. The first fraction (DEAE pl and p2) eluted from the column containing MIS activity is dialyzed against phosphate buffer at about pH 6.0 overnight and concentrated to about15 ml (with a protein eoncentration of about 4 mg/ml). DEAE pl and p2 fractions are combined and fractionated through a CM Bio-Gel A column equilibrated with phosphate buffer, about pH 6.5. The first fraction (CM pl) eluted from the column containing AMH activity is collected, concentrated, and dialyzed against Buffer A (containing about 10 mM N-2-hydroxyethyl-piperazine-N′-ethanesulfonic acid (HEPES), about 150 mM NaCl, about 5 mM 2-mercaptoethanol, about 1 mM EDTA, and about 0.01% Nonidet-P40 with pH 7.0). The CM pl fraction is loaded onto a wheat germ lectin (WGL) Sepharose 6B column (1.5 X 16 cm) and eluted with Buffer A until the unbound fraction is completely eluted. The bound fraction is removed by the addition of about 100 mg/ml N-acetyl-D-glucosamine in Buffer A. The WGL column retained the fraction (WGL p2) containig AMH activity. This fraction is concentrated with an Amicon cell and dialyzed against phosphate buffer (containing about 10 mM sodium phosphate, about 0.15 M NaCl, and about 1 mM. EDTA, pH about 7.0). Finally, the WGL p2 fraction is dissolved in about 0.1% (v/v) trifluoroacetic acid and is further purified by a high performance liquid column chromatographic system. The AMH active protein has a molecular mass of about 74,000 Kd and has a pl of about 6.1

In vitro biological activity of avian AMH is determined by mullerian duct culture regression. Groups of Mullerian ducts (two ducts per group) are each incubated in a Falcon 3010 organ culture dish on a stainless steel grid supported with triangular-shaped Whatman No. 2 filter paper. The inner well contained modified Dulbecco's medium (1.2 ml/well) with penicillin (10,000 units/ml) and streptomycin (10,000 units/ml). The surrounding absorbent pad was filled with glass-distilled water. The test material i.e., the crude or purified MIS preparation, is dissolved in the modified Dulbeceo's medium by dialyzation and was placed around the Mullerian duct. Cultures are incubated for about 72 hr at about 37°C in about 5% $CO_2$ and about 95% air in a New Brunswick incubator (Model CO-20). Under these conditions, Mds can survive for about 7 to about 10 days if AMH is not added. The degrees of Mullerian duct regression induced by these protein fractions have been defined and graded as follows: (1) complete regression with no remnants; (2) subtotal regression with thin cranial remnants; (3) incomplete regression with partial cranial and caudal remnants, (4) atrophic ducts with thin and underdeveloped length; (5) slight atrophy in the cranial region. The culture of the embryonic intestine and Wolffian ducts followed the same technique.

Fertilized poultry embryos, preferably chicken turkey, duck or goose, are treated on about day 0 to about day 9 of incubation with one or more aromatase inhibitors plus AMH. Preincubation as used herein is considered to be day 0, thus day 1 is the day the eggs are placed in the incubator. Incubation begins when the embryos are placed in an incubator. Treatment is by one or more injections of the aromatase inhibitors in combination with AMH into the embryo or by dipping the embryonated eggs one or more times in a solution of the aromatase inhibitors. A single injection is given on about day 0 to about day 9 of incubation. Multiple injections are given every other or every third day starting at about day 0 of incubation and are continued through about day 9 of incubation. Dipping of embryos is by a single treatment or multiple treatments at times discussed above. Dipping as used herein also includes pressure impregnating fertilized eggs with one or more aromatase inhibitors. The aromatase inhibitor or inhibitors are dissolved or suspended in a physiologically acceptable carrier. Such aqueous physiologically acceptable carriers include, but are not limited to water, saline, physiologic saline, buffer saline, phosphate buffered saline, phosphate buffered saline glucose. Non-aqueous physiologically acceptable carriers include, but are not limited to, 1,2-propanediol, dimethyl sulfoxide, ethanol and the like. The concentration of the aromatase inhibitor or inhibitors is from about 0.1 mg/ml to about 100 mg/ml and the injection volume ranges from about 10 μl to about 100 μl producing doses of about 1 μg to about 10 mg. The concentration of AMH is from about 10 μg/ml to about 10 mg/ml and the injection volume ranges form about 10 μl to about 100 μl providing doses of about 0.1 μg to about 1 mg. Injections are made by hand or by machine with about 23 G by 1 in needles. The injection is made under the air sac into the egg white surrounding the developing embryo. Administration of the aromatase inhibitor plus AMH is also possible by injections made into the air sac without piercing the membrane. Administration is also possible by injection into the opposite end of the egg directly into the albumin. The injection hole is sealed with Scotch tape, wax, glue and the like. The aromatase inhibitor plus AMH can also be administered by dipping the egg in a solution containing one or more inhibitors at concentrations as described above. Injected or dipped eggs are placed in a humidified, rocking incubator and allowed to develop and hatch.

Conversion to male phenotype is determined at about day 14 of incubation, at hatching and post-hatching. Pre-hatching conversion is determined by removing the embryos from their shells and dissecting to observe development of the gonads. Male phenotype is determined following hatching by organ sex characterization and/or by vent sexing which are known in the art. The conversion of the genotypic female poultry to the phenotypic male poultry results in secondary sex characteristics such as increased weight gain and/or an improved lean to fat ratio.

The inhibition of aromatase activity prior to about day 9 of embryogenesis triggers a developmental switch which causes the indifferent gonad of the genetically female embryo to differentiate into a testis rather than an ovary. The present invention is unique in that a single treatment which results in the inhibition of aromatase activity results in an irreversible change in the normal program for sexual differentiation. This treatment results in a female chicken developing a male phenotype. The irreversible nature of the reversal is illustrated by the fact that the genotypic female has all the behavioral characteristics of males. Most significant is the fact that upon attaining sexual maturity, which occurs at about seven months after a single treatment with an aromatase inhibitor in combination with AMH , the testes of the genetic female produce sperm.

A major advantage of treating developing embryos with active aromatase inhibitors plus AMH is the inhibition of the conversion of androgens to estrogens which normally takes place in developing female poultry and allows gonadal development. This inhibition results in an increase in the ratio of androgens to estrogens which induces or converts the embryo to the male phenotype without having to treat the embryos with androgens or other hormones.

The following examples illustrate the present invention without, however, limiting the same thereto.

EXEMPLE 1

Use Of Anti-Mullerian Hormone For The Reversal Of Female Sexual Phenotype In Poultry

Fertilized eggs from a broiler line of chickens (Arbor Acre X Arbor Acre ) are injected with a solution containing purified chicken anti-Mullerian hormone (AMH). The solution is prepared fresh by carefully dissolving the purified AMH in phosphate buffered saline at concentrations ranging from 0.05 mg/ml to 5 mg/ml. Each egg is injected with 0.l ml of the solution using an appropriate syringe fitted with a 23G X 1 inch needle. The injection is made on day 5 or 6 of incubation and in the large end of the egg just underneath the air sac. The injection hole is sealed with Scotch tape and the eggs are placed back into a humidified, rocking incubator. All chicks are vent sexed after hatching. Treatment with AMH causes the genetic females to permanently adopt a male phenotype. Thus, genetic females vent sex as males and in time develop the external appearance and behavior of true genetic males. Furthermore, these sex-reversed males have two testes capable of producing sperm. Performance characteristics such as increased rate of weight gain and improved lean to fat ratio that are associated with males are also observed.

EXAMPLE 2

Use Of Anti-Mullerian Hormone In Combination with Aromatase Inhibiotrs For The Reversal Of Female Sexula Phenotype In Poultry

Fertilized eggs from a broiler line of chickens (Arbor Acre X Arbor Acre ) are injected with a solution containing purified chicken anti-Mullerian hormone (AMH) and the aromatase inhibitor ±5-(p-cyanophenl)-5-6-7-8-tetrahydroimidazo[l,5-α]pyridine hydrochloride (AI). The solution is prepared fresh by carefully dissolving the purified AMH in phosphate buffered saline at a concentration of about l mg/ml. AI is dissolved at a concentration of about l0 mg/ml. Each egg is injected with 0.lml of the solution using an appropriate syringe fitted with a 23G X 1 inch needle. The injection is made on about day 5 of incubation and in the large end of the egg just underneath the air sac. The injection hole is sealed with Scotch tape and the eggs are placed back into a humidified, rocking incubator. All chicks are vent sexed after hatching. Treatment with AMH in combination with an aromatase inhibitor causes the genetic females to permanently adopt a male phenotype. Thus, genetic females vent sex as males and in time develop the external appearance and behavior of true genetic males. Furthermore, these sex- reversed males have two testes capable of producing sperm. Performance characteristics such as increased rate of weight gain and improved lean to fat ratio that are associated with males are also observed.

## Claims

1. A method for converting genotypic female poultry into phenotypic male poultry comprising administering to a healthy poultry embryo a phenotypic converting amount of a substance which prevents the conversion of androgens to estrogens in combination with fowl anti-mullerian hormone which results in the conversion of genotypic female poultry into phenotypic male poultry.

2. A method for converting genotypic female poultry into phenotypic male poultry comprising administering to a healthy poultry embryo a phenotypic converting amount of a substance which prevents the conversion of androgens to estrogens and causes mullerian duct regression and results in the conversion of genotypic female poultry into phenotypic male poultry.

3. A method for converting genotypic female poultry into phenotypic male poultry comprising administering to a healthy poultry embryo a phenotypic converting amount of a substance which prevents the conversion of testosterone or androstenedione to estradiol or estrone in combination with fowl anti-mullerian hormone which results in the conversion of genotypic female poultry into phenotypic male poultry.

4. A method for converting genotypic female poultry into phenotypic male poultry comprising administering to a healthy poultry embryo a phenotypic converting amount of fowl antimullerian hormone which results in the conversion of genotypic female poultry into phenotypic male poultry.

5. A method for converting genotypic female poultry into phenotypic male poultry comprising administering to a healthy poultry embryo a phenotypic converting amount of an aromatase inhibitor in combination with fowl anti-mullerian hormone.

6. Phenotypically male poultry comprising genotypic females treated with one or more aromatase inhibitors in combination with fowl anti-mullerian hormone which converts the females to a male phenotype.

7. Phenotypically male poultry comprising genotypic females treated with fowl anti-mullerian hormone which converts the females to a male phenotype.

8. Genotypically female poultry having a male phenotype resulting from treating the developing embryo with one or more aromatase inhibitor in combination with fowl anti-mullerian hormone.

9. Genotypically female poultry having a male phenotype resulting from treating the developing embryo with fowl antimullerian hormone.

10. A composition for converting genotypic female poultry into phenotypic male poultry comprising administering to a healthy poultry embryo a phenotypic converting amount of a substance which prevents the conversion of androgens to estrogens in combination with fowl anti-mullerian hormone which results in the conversion of genotypic female poultry into phenotypic male poultry.

11. A composition for converting genotypic female poultry into phenotypic male poultry comprising administering to a healthy poultry embryo a phenotypic converting amount of fowl anti-mullerian hormone which results in the conversion of genotypic female poultry into phenotypic male poultry.

12. The aromatase inhibitors according to claim 5, 6 or 8 wherein the said aromatase inhibitor is a steroid substrate analogue or a non-steroid analogue which binds and inactivates aromatase or is a compound that inhibits the cytochrome P450 component of the aromatase complex.

13. A method for increasing weight gain and/or an improved lean to fat ratio in genotypic female poultry comprising treatment of geontypic female poultry embryos with one or more aromatase inhibitors in combination with fowl anti-mullerian hormone.

14. A method for increasing weight gain and/or an improved lean to fat ratio in genotypic female poultry comprising treatment of genotypic female poultry embryos with fowl anit-mullerian hormone.

15. The aromatase inhibitor according to claim 5, 6 or 8 wherein the said aromatase inhibitor is selected from the group consisting of: 6-[(1H-imidazol-1-yl)phenylmethyl]-1 methyl-1H-benzotriazole, 6-[(4-chlorophenyl)(1H-1,2,4-triazol-1-yl)methyl] 1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, and 2,2-[5-dideuterio(1H-1,2,4-triazol-1-yl)methyl-1,3-phenylene]di(2trideuteriomethyl-3,3,3-(trideuteriopropiononitrile), and 2,2-[5-dideuterio(1H-1,2,4-triazol-1-ylmethyl-1, 3-phenylene)di(2-methylpropiononitrile); 1,1-dimethyl-8-(1H-1,2,4-triazol-1-ylmethyl)-2(1H)-naphtho[2,1-b]furanone, 1,2-dihydro-1,1-dimethyl-2-oxo-8-(1H-1,2,4-triazol-1-ylmethyl)naphtho-[2,1-b] furan-7-carbonitrile, 1,2-dihydro-1,1-dimethyl-2-oxo-8-(1H-1,2,4-triazol-1-ylmethyl)naphtho[2,1-b] furan-7-carboxamide, and 1,2-dihydro-1,1-dimethyl-2-oxo-8-[di(1H-1,2,4-triazol-1yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)-1-(1,2,4-triazol-1-yl)2-(1,2,4-triazol-1-ylmethyl)butan-2-ol, 2-(4-chloro-α-fluorobenzyl)-1,3-di(1,2,4-triazol-1-yl)propan-2-ol, 2-(4-chlorobenzyl)1,3-bis(1,2,4-triazol-1-yl)propane, 4-[2-(4-chlorophenyl)-1,3-di(1,2,4-triazol-1-ylmethyl)ethoxymethyl]-benzonitrile, 1-(4-fluorobenzyl)-2-(2-fluoro-4-trifluoromethylphenyl)-1,3-di(1, 2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)-2-(2,4-difluorophenyl)-1,3-di(1,2,4-triazol-1-yl)-propan-2-ol and 2-(4-chlorophenyl)-1-phenyl-1,3-di(1,2,4-triazol-1-yl) propan-2-ol; 5-bis(4-chlorophenyl)methylpyrimidine; α,α-bis(4-chlorophenyl)-2-pyrazinemethanol; N-(2,4-difluorophenyl)-N-benzyl-3-pyridinemethanamine and N-(2-chlorophenyl-α-(4-fluorophenyl)-3-pyridinemethanamine; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and a,a-bis(4-chlorophenyl)-3-pyridinemethanol; 5H-5-(4-cyanophenyl)-6,7-dihydropyrrolo[1,2-c]imidazole and 5H-5-(4-cyanophenyl)-6,7,8,9-tetrahydroimidazo [1,5-a]azepine; 5-[(1 H-imidazol-1-yl)phenylmethyl]-2-methyl-1H-benzimidazole and 5-[(3-chlorophenyl)(1H-imidazol-1-yl)-methyl]-1 H-benzimidazole; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl) stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)stilbene-4-carbonitrile, (Z)-α-(imidazol-1-ylmethyl) stilbene4,4'-dicarbonitrile,(Z)-α-(5-methylimidazol-1-ylmethyl)stilbene-4,4'-dicarbonitrile, and (Z)-2-[2-(4-cyanophenyl)-3-(1,2,4-triazol-1-yl)propenyl] pyridine-5-carbonitrile; (1R* ,2R*)-6-fluoro-2-(4-fluorophenyl)-1,2,3,4-tetrahydro-1-(1H-1,2,4-triazol-1-ylmethyl)naphthalene, (1R* ,2R*)-6-fluoro-2-(4-fluorophenyl)-1,2,3,4-tetrahydro-1-(1H-imidazolylmethyl)-naphthalene, (1R* ,2R*)-and (1R* , 2S*)-2-(4-fluorophenyl)-1,2,3,4-tetrahydro-1-(1H-1,2,4-triazol-1-ylmethyl)naphthalene-6-carbonitrile, (1R* ,2R*)-and  (1R* ,2S*)-2-(4-fluorophenyl)-1, 2,3,4-thetahydro-1-(1H-imidazolylmethyl)naphthalene-6-carbonitrile, (1R* ,2R*)-and (1R* ,2S*)-1, 2,3,4-tetrahydro-1-(1H-1,2,4-triazol-1-ylmethyl) naphthalene-2,6-dicarbonitrile, (1R* ,2S*)-and (1R* ,2S*)-1,2,3,4-tetrahydro-1-(1H-imidazol-1-ylmethyl)naphthalene-2,6-dicarbonitrile, and(1R* , 2S*)-2-(4-fluorophenyl)-1,2,3,4-tetrahydro-1-(5-methyl-1H-imidazolylmethyl)naphthalene-6-carbon itrile;  8-chloro-5-(4-chlorophenyl)-5H-indeno[1, 2-d]pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3,17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3,17-dione; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole; 10β-thiiranylestr-4-ene-3, 17-dione and 10β-oxiranylestr-4-ene-3, 17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)-2(1H)-naphtho [2, 1-b]furanone (1a); ± 5- (p-cyanophenyl)-5, 6, 7, 8-tetrahydroimidazo [1, 5-a] pyridine hydrochloride; 1,4,6-androstatriene-3,17-dione; and bis-(p-cyanophenyl)-imidazo-1-yl-methane hemisuccinate and pharmaceutically acceptable deritives, acid addition salts thereof

16. The aromatase inhibitor of claim 15 wherein said aromatase inhibitor is ± 5- (p-cyanophenyl)-5, 6, 7, 8-tetrahydroimidazo [1, 5-α] pyridine hydrochloride.

17. The aromatase inhibitor of claim 16 wherein said aromatase inhibitor is 1,4,6-androstatriene-3,17-dione.

18. The method according to claim 2 or 4 wherein the poultry embryos are treated with fowl anti-mullerian hormone on about day 0 to about day 9 of incubation.

19. The method according to claim 1 or 3 wherein the poultry embryos are treated with the aromatase inhibitor plus fowl anti-mullerian hormone on about day 0 to about day 9 of incubation.

| European Patent Office | EUROPEAN SEARCH REPORT | Application Number |
|---|---|---|

EP    92 30 3689

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-0 433 084 (MERCK & CO. INC) 10 June 1991<br><br>* claims 11,12,15-18 *<br>--- | 6-9,12,<br>15-17 | A61K37/24<br>A61K31/00<br>A61K31/565<br>A61K31/44 |
| P,Y | SCIENCE<br>vol. 255, no. 5043, 24 January  1992,<br>pages 467 - 470;<br>A.ELBRECHT ET AL: 'Aromatase Enzyme Activity and Sex Determination in Chickens'<br>*Summary*<br>--- | 1-5,10,<br>11,13,<br>14,18,19 | |
| Y | BULL. ASSOC.ANAT.<br>vol. 75, no. 225, 1991,<br>pages 21 - 28;<br>R.MARAUD ET AL: 'Apport de l'intersexualité expérimentale chez le Poulet au problème de la différentiation et de ses anomalies chez les vertébrés amniotes'<br>*summary; page 25, right column, paragraph 4*<br>--- | 1-5,10,<br>11,13,<br>14,18,19 | |
| D,Y | GEN. COMP. ENDOCRINOL.<br>vol. 57, no. 1, 1985,<br>pages 88 - 102;<br>J.M.HUTSON ET AL.: 'Steroid Modulation of Mullerian Duct Regression in the Chick Embryo'<br>*abstract*<br><br>----- | 1-5,10,<br>11,13,<br>14,18,19 | TECHNICAL FIELDS SEARCHED (Int. Cl.5 )<br><br>A61K |

The present search report has been drawn up for all claims

| Place of search | Date of completion of the search | Examiner |
|---|---|---|
| MUNICH | 07 AUGUST 1992 | TZSCHOPPE O.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................

& : member of the same patent family, corresponding document

EPO FORM 1503 03.82 (P0401)